(19) **Europäisches Patentamt**

**European Patent Office**

**Office européen des brevets**

(11) **EP 1 172 641 A1**

(12) **EUROPEAN PATENT APPLICATION**
published in accordance with Art. 158(3) EPC

(43) Date of publication:
**16.01.2002   Bulletin 2002/03**

(51) Int Cl.[7]: **G01N 19/00**, G01N 11/00,
G01N 33/44, C08L 21/00,
C08J 3/20, C08C 4/00

(21) Application number: **01902682.2**

(22) Date of filing: **31.01.2001**

(86) International application number:
**PCT/JP01/00634**

(87) International publication number:
**WO 01/57493 (09.08.2001 Gazette 2001/32)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **31.01.2000  JP  2000027367**

(71) Applicant: **Mitsui Chemicals, Inc.
Tokyo 100-6070 (JP)**

(72) Inventors:
• **Nakahama, Hidenari c/o Mitsui Chemicals, Inc.
Ichihara-shi, Chiba 299-0108 (JP)**
• **Kawasaki, Masaaki c/o Mitsui Chemicals, Inc.
Ichihara-shi, Chiba 299-0108 (JP)**

(74) Representative: **Nicholls, Michael John et al
J.A. KEMP & CO. 14, South Square Gray's Inn
London WC1R 5JJ (GB)**

(54) **METHOD OF TESTING RUBBER COMPOSITION FOR KNEADED STATE AND PROCESS FOR PRODUCING RUBBER COMPOSITION**

(57)   A kneading status evaluation method for a rubber composition containing at least a rubber and a filler comprises the steps of a complex modulus measurement step (1) in which a complex modulus E* (a) at a given strain $\varepsilon$ a and a complex modulus E*(b) at a given strain $\varepsilon$ b differing from the strain $\varepsilon$ a of the rubber composition (I) are measured, a filler dispersion index calculation step (2) in which a filler dispersion index (N) of the rubber composition (I) is calculated with complex elastic moduli E* (a) and E* (b) obtained in the previous step (1) according to the equation shown below, and a comparison step (3) to compare a predetermined target filler dispersion index (R) with the filler dispersion index (N) calculated in the previous step (2), and/or a complex viscosity coefficient measurement step (5) to measure a complex viscosity coefficient $\eta$* of the rubber composition (I) under at least two different temperatures, and a kneading status monitor index calculation step (6) to calculate a kneading status monitor index (M) of the rubber composition (I) according to the equation shown below on the basis of a temperature dependency of the complex viscosity coefficient $\eta$ * obtained at the previous step (5), and a comparison step (7) to compare a predetermined target kneading status monitor index (P) with the kneading status monitor index (M) calculated in the previous step (6);

$$\text{Filler dispersion index (N)} = |E^*(a)|/|E^*(b)|$$

$$|\eta\ ^*(T)| = A \exp (-M/ RT)$$

where $\eta$ *: complex viscosity coefficient, A: proportional constant, R: gas constant, and T: measuring temperature (°K).

A manufacturing method for a rubber composition is characterized by carrying out the evaluation methods described above.

Implementation of the evaluation methods described above makes it possible to evaluate objectively a kneading status of a rubber composition containig at least a rubber and a filler. Further, implementation of the manufacturing methods described above can provide a rubber composition having good filler dispersion and a stable kneading status.

EP 1 172 641 A1

**Description**

**Technical Field of the Invention**

The present invention relates to evaluation methods for kneading status of a rubber composition and manufacturing methods for a rubber composition, for example, a manufacturing method for an ethylene-α-olefin based copolymer rubber composition for cross-linking which has good filler dispersion and a stable kneading status, more specifically in which at least an ethylene-α-olefin based copolymer rubber and a reinforcing filler such as a carbon black, specifically 30 parts by weight or more of a carbon black per 100 parts by weight of the ethylene-α-olefin based copolymer rubber, are kneaded by a closed type mixer, and then the resultant kneaded material is compounded with a vulcanizing agent or a cross-linking agent and a vulcanization accelerator or a cross-linking aid.

**Technical background for the Invention**

Quality of a rubber product is greatly influenced by a rubber compounding technology. Particularly, an ethylene-α -olefin based copolymer rubber such as EPR and EPDM has no mechanical strength by itself and thus requires a large amount of a reinforcing filler such as a carbon black.

However, in the case of dispersing such a filler into an ethylene-α-olefin based copolymer rubber, viscosity of a rubber like an ethylene-α-olefin based copolymer rubber will be generally higher than that of a plastic resin, and thus it becomes a very difficult technique to finely disperse the filler into the ethylene-α-olefin based copolymer rubber.

Hence, in the case of kneading an ethylene-α-olefin based ting copolymer rubber with a filler, a method of applying a large shear stress or prolonging a kneading time is conventionally adopted to achieve a fine filler dispersion, and then kneading conditions are adjusted by selecting more efficient kneading conditions of them with respect to product properties and processability in order to avoid lowering investment cost-performance and productivity.

However, there has been no definitive index for a kneading status and filler dispersion, and therefore, it is common at the present day that kneading conditions are decided according to arbitral standards.

Further, it has been known that a kneading status of an ethylene-α-olefin based copolymer with a filler varies depending on quite a lot of variation in atmospheric temperature and humidity season by season. Because there has been no simple index available to analyze such a kneading status change so far, there were many cases in which a proper solution could not be applied at a production site even if there happened phenomena such as a change in a sectional shape (die swell) of extrusion products and a frequent appearance of bubbles on products by unknown reasons. In such cases, the defects described above were often caused by an occasional combination of weather conditions such as temperature and humidity and kneading specifications of a mixer (shear stress, dispersion rate), and therefore there were many cases that the defects could not be observed anymore after some period of time passed or by use of a different mixer even with the same formulation. In fact, it has been the present status that insufficient analysis is carried out because it is very difficult to detect a cause.

As a conventional method to evaluate filler dispersion, an electric resistance measurement method, a microscope method, and a light reflection method basing on a degree of light reflected from a rubber compound surface are known so far. However, a use of these methods as a monitoring index for detecting the defects described above is not sufficient because values given by these methods are subject to influence of a water content, a molecular weight distribution of polymer and so on, so that a definitive result can not be obtained even though the values are changeable somehow according to a variation of filler dispersion.

In addition, an ethylene-α-olefin based copolymer rubber such as EPR and EPDM is a non-polar polymer, and thus, when it is kneaded with a carbon black which has a nature of polarity, it has been known that a die swell changes more and physical properties become worse through a progress of kneading by a closed type mixer. This fact is generally known as a pseudo-gel phenomenon and it can be scarcely observed in ML (1+4) Mooney viscosity at 100°C. However, when it is continuously rotated for about 1 hour, i.e., ML (1+59) 100°C, a phenomenon with a large increase of the Mooney viscosity (torque) can be easily observed during the measurement time. A status under rotation in a Mooney viscometer is analogous to an inside status of a barrel of an extruder or a status of a pot of an injection molding machine, whicharerubberprocessingmachines, and the phenomenon will take place in spite of no content of vulcanizing agents or cross-linking agents. Therefore, another index for monitoring a kneading status rather than conventional indexes for filler dispersion has been required for a rubber composition in which an ethylene-α-olefin based copolymer rubber and a carbon black are compounded.

As a matter of fact, as an analytical method capable to provide both a filler dispersion index and a kneading status monitor index at high accuracy, the wide range NMR method (Kiuchi Yasutaro and Ito Masayoshi: Japan Rubber Associate Magazine, 72, 1999) has been known. However, because of its high analysis cost and a slow response for an evaluation result, this method is not appropriate to use as an analytical method for quality control in a factory.

Consequently, appearances of evaluation methods for kneading status of a rubber composition which can provide an objective evaluation for kneading status of a rubber composition containing at least a rubber and a filler, and of manufacturing methods of a rubber composition by using the evaluation methods are desired in order to achieve preferable filler dispersion and a stable kneading status. More specifically, it is desired to find new analytical indexes which enable to evaluate objectively, for example, filler dispersion and a kneading status of an ethylene-$\alpha$-olefin based copolymer rubber composition for cross-linking obtained by a closed type mixer, and then, it is desired to find new manufacturing methods for an ethylene-$\alpha$-olefin based copolymer rubber composition for cross-linking which has good filler dispersion and a stable kneading status by means of kneading an ethylene-$\alpha$-olefin based copolymer rubber and a reinforcing filler such as a carbon black by using a closed type mixer, and then of kneading the resultant kneaded material with a vulcanizing agent or a cross-linking agent and the vulcanization accelerator or the cross-linking aid by using a mixer such as an 8-inch open roll mill.

The present invention has a purpose of solving the problems described above which inherent in conventional compounding technologies, and of providing evaluation methods to evaluate objectively a kneading status of a rubber composition containing at least a rubber and a filler.

Further, the present invention has a purpose of providing manufacturing methods for a rubber composition having good filler dispersion and a stable kneading status by adopting the evaluation methods described above.


**DESCLOSURE OF THE INVENTION**


The first kneading status evaluation method for a rubber composition of the present invention is a kneading status evaluation method for a rubber composition (I) containing at least a rubber (A) and a filler (B), which comprises the steps of;

(1) a complex modulus measurement step to measure a complex modulus of E*(a) at a given strain E a and a complex modulus E*(b) at a given strain $\varepsilon$ b differing from the strain $\varepsilon$ a;

(2) a filler dispersion index calculation step to calculate a filler dispersion index (N) of the rubber composition (I) according to the following equation;

$$\text{Filler dispersion index (N)} = |E^*(a)|/|E^*(b)|$$

where the complex moduli E*(a) and E*(b) are obtained at the complex modulus measurement step (1) ; and

(3) a comparison step to compare a predetermined target filler dispersion index (R) with the filler dispersion index (N) calculated in the filler dispersion index calculation step (2).


The target filler dispersion index (R) is generally obtained as a target value (NO) of a filler dispersion index, through the complex modulus measurement step (1) and the filler dispersion index calculation step (2), after a rubber composition having the same formulation as that of the rubber composition (I) is substantially kneaded to achieve practically complete dispersion.

The practically complete dispersion is preferably achieved by kneading with an open roll mill.

The following evaluation method can be adopted as a simple alternate of the first evaluation method for kneading status of a rubber composition of the present invention.

That is, this simple alternate is a kneading status evaluation method for a rubber composition (I) containing at least a rubber (A) and a filler (B), which comprises the steps of;

(1') a dynamic elastic modulus measurement step to measure a dynamic elastic modulus E' (a) at a given strain $\varepsilon$ a and a dynamic elastic modulus E' (b) at a given strain $\varepsilon$ b differing from the strain $\varepsilon$ a of a crosslinked rubber sheet obtained by crosslinking the rubber composition (I);

(2') a filler dispersion index calculation step to calculate a filler dispersion index (N') of the rubber composition (I) according to the following equation;

$$\text{Filler dispersion index (N')} = E'(a) / E'(b)$$

where the dynamic elastic moduli E'(a) and E'(b) are obtained at the dynamic elastic modulus measurement step (1'); and

(3') a comparison step to compare a predetermined target filler dispersion index (R') with the filler dispersion index (N') calculated in the filler dispersion index calculation step (2').

The target filler dispersion index (R') is generally obtained as a target value (NO') of a filler dispersion index, through the dynamic elastic modulus measurement step (1') and the filler dispersion index calculation step (2'), after a rubber composition having the same formulation as that of the rubber composition (I) is substantially kneaded to achieve practically complete dispersion.

The practical complete dispersion is preferably achieved by kneading with an open roll mill.

The dynamic elastic modulus E' corresponds to a real part of complex modulus E* and can be formulated as the following equation;

$$E^* = E' + iE''.$$

The real part E' is about ten times bigger than the imaginary part E'' regarding a general rubber, so that a ratio of real part E' itself becomes nearly equal to a ratio of absolute value of complex modulus E* itself. Thus, the kneading status evaluation method adopting dynamic elastic modulus E' can simplify a calculation at the filler dispersion index calculation step, and it is superior regarding this point. In addition, the measurement of dynamic elastic modulus E' can be done in the same instrument and by the same measurement method for complex modulus E* as described later.

The first manufacturing method for a rubber composition of the present invention is characterized by utilizing the first evaluation method (including the simple alternate)for kneading status of a rubber composition of the present invention as described above.

This manufacturing method, in general, further comprises a feedback step (4) or (4') to control kneading conditions of the rubber composition (I) by means of adjusting a value of filler dispersion index (N)/target filler dispersion index (R) to be a certain numeric range, or a value of filler dispersion index (N') /target filler dispersion index (R') to be a certain numeric range, according to the result from the comparison step (3) or (3').

The numeric range of the value of filler dispersion index (N)/target filler dispersion index (R) (where |E*(a) |≦|E*(b) |) or the value of filler dispersion index (N')/target filler dispersion index (R') is preferably 0.8 to 1.0.

The second evaluation method for kneading status of the present invention is a kneading status evaluation method for a rubber composition (I) containing at least a rubber (A) and a filler (B), which comprises the steps of:

(5) a complex viscosity coefficient measurement step to measure a complex viscosity coefficient η * of the rubber composition (I) under at least two different temperatures;

(6) a kneading status monitor index calculation step to calculate a kneading status monitor index (M) of the rubber composition (I) according to the following equation;

$$| \eta * (T)| = A \exp (-M/ RT)$$

(where η *: complex viscosity coefficient, A: proportional constant, R: gas constant, and T: measuring temperature (°K)), that shows a temperature dependency of the complex viscosity coefficient η * obtained at the complex viscosity coefficient measurement step (5); and

(7) a comparison step to compare a predetermined target kneading status monitor index (P) with the kneading status monitor index (M) calculated in the kneading status monitor index calculation step (6).

The target kneading status monitor index (P) is generally obtained as a target value (M0) of a kneading status monitor index, through the complex viscosity coefficient measurement step (5) and the kneading status monitor index calculation step (6), after a rubber composition having the same formulation as that of the rubber composition (I) is substantially kneaded to achieve practically complete dispersion.

The practically complete dispersion is preferably achieved by kneading with an open roll mill.

The following evaluation method can be adopted as a simple alternate of the second evaluation method for kneading status of a rubber composition of the present invention.

That is, this simple alternate is a kneading status evaluation method for a rubber composition (I) containing at least a rubber (A) and a filler (B), which comprises the steps of;

(5') a complex viscosity coefficient measurement step to measure a complex viscosity coefficient η' as a real part of complex viscosity coefficient η * of the rubber composition (I) under at least two different temperatures;

(6') a kneading status monitor index calculation step to calculate a kneading status monitor index (M') of the rubber composition (I) according to the following equation;

$$\eta\,'(T) = A \exp (-M'/ RT)$$

(where A: proportional constant, R: gas constant, and T: measuring temperature ($^\circ$K)), that shows a temperature dependency of a real viscosity coefficient $\eta'$ obtained as a real part of complex viscosity coefficient $\eta *$ at the complex viscosity coefficient measurement step (5'); and

(7') a comparison step to compare a predetermined target kneading status monitor index (P') with the kneading status monitor index (M') calculated in the kneading status monitor index calculation step (6').

The target kneading status monitor index (P') is generally obtained as a target value (M0') of a kneading status monitor index, through the complex viscosity coefficient measurement step (5') and the kneading status monitor index calculation step (6'), after a rubber composition having the same formulation as that of the rubber composition (I) is substantially kneaded to achieve practically complete dispersion. The practical complete dispersion is preferably achieved by kneading with an open roll mill.

The real viscosity coefficient $\eta'$ corresponds to a real part of complex viscosity coefficient $\eta *$ and can be formulated as the following equation;

$$\eta* = \eta' + i\eta''$$

The real part $\eta'$ is about ten times bigger than an imaginary part $\eta''$ regarding a general rubber, therefore, when a so-called Arrhenius plot of a real part $\eta'$ is drawn, the plot becomes very close to an Arrhenius plot of absolute values of the complex viscosity coefficient $\eta*$. Thus, the kneading status evaluation method adopting a real part $\eta'$ of complex viscosity coefficient $\eta *$ can simplify a calculation at the kneading status monitor index calculation step, and it is superior in this point. In addition the measurement of a real part $\eta'$ of complex viscosity coefficient $\eta*$ can be done in the same instrument and by the same measurement method for a complex viscosity coefficient $\eta *$ as described later.

The second manufacturing method for a rubber composition of the present invention is characterized by utilizing the second evaluation method (including the simple alternate) for kneading status of a rubber composition of the present invention as described above.

This second manufacturing method, in general, further comprises a feedback step (8) or (8') to control kneading conditions for the rubber composition (I) by means of adjusting a value of kneading status monitor index (M)/target kneading status monitor index (P) to be a certain numeric range, or a value of kneading status monitor index (M')/target kneading status monitor index (P') to be a certain numeric range, according to the result from the comparison step (7) or (7').

The numeric range of the value of kneading status monitor index (M)/target kneading status monitor index (P) or the value of a kneading status monitor index (M')/target kneading status monitor index (P') is preferably 0.85 to 1.0

In addition, the first manufacturing method for a rubber composition of the present invention may comprise the steps (from (5) to (7), and (8)) of the second manufacturing method for a rubber composition of the present invention. On the contrary, the second manufacturing method for a rubber composition of the present invention may comprise the steps (from (1) to (3), and (4)) of the first manufacturing method for a rubber composition of the present invention.

The present invention can be implemented in various applications according to a kind, nature, and usage of a rubber composition, and however, the following practical application can be shown as a preferred example of the manufacturing methods for a rubber composition of the present invention. For example, in the case of a manufacturing method for an ethylene-$\alpha$-olefin based copolymer rubber composition for cross-linking in which at least an ethylene-$\alpha$-olefin based copolymer rubber and a reinforcing filler; specifically 30 parts by weight or more of a reinforcing filler per 100 parts by weight of the ethylene-$\alpha$-olefin based copolymer rubber, are kneaded by a closed type mixer and compounded with a vulcanizing agent or a cross-linking agent and, if necessary, a vulcanization accelerator or a cross-linking aid, after a filler dispersion index (R) and/or a kneading status monitor index (P) are predetermined according to the analytical methods described below, kneading conditions of a closed type mixer is controlled such that a filler dispersion index (N) obtained by the analytical method described below satisfies the following equation;

Filler dispersion index (N) / filler dispersion index (R) = 1 to 0.8,

and/or such that a kneading status monitor index (M) obtained by the analytical method described below satisfies the following equation;

Kneading status monitor index (M) / kneading status monitor index (P) = 1 to

0.85

(1) Reference filler dispersion index (R) : After measuring strain dependency of a dynamic elastic modulus of an even thickness cross-linkable rubber sheet which is formed from an unvulcanized (uncross-linked) rubber composition obtained by kneading at least an ethylene- $\alpha$-olefin based copolymer rubber, a reinforcing filler, a vulcanizing agent or a cross-linking agent, and if necessary, a vulcanization accelerator or a cross-linking aid at a temperature of 100°C or lower by using an 8-inch open roll mill, then a reference filler dispersion index (R) is calculated as a percentage of ( (E*(b)/E*(a)) x 100) (more precisely (|E*(b)|/|E*(a)|) x100) where E* (a) isadynamic elastic modulus (more precisely a complex modulus) at a given strain in a zone where a dynamic elastic modulus (more precisely a complex modulus) had little change against a strain change, and E*(b) is a dynamic elastic modulus (more precisely a complex modulus) at a given strain in a zone where a dynamic elastic modulus (more precisely a complex modulus) had a large change against a strain change.

Noticing that "reference filler dispersion index (R) "used in this practical example corresponds to "target filler dispersion index (R)" used in the first evaluation method of the present invention, and also corresponds to the target value (NO) of filler dispersion index used in the first evaluation method of the present invention.

(2) Reference kneading status monitor index (P) : Temperature dependency of a complex viscosity coefficient of an uncured rubber composition obtained by kneading at least an ethylene-$\alpha$-olefin based copolymer rubber with a reinforcing filler at a temperature of 100°C or lower by using an 8-inch open roll mill without any vulcanizing agent, any cross-linking agent, any vulcanization accelerator, nor any cross-linkingaidis shown in the following equation;

$$\eta^* = A \exp(-Ea / RT)$$

(where $\eta^*$: complex viscosity coefficient, Ea: apparent activation energy, T: measuring temperature (°K), R: gas constant, and A: proportional constant), or

$$a_T = A \exp(-Ea / RT)$$

(where $a_T$: shift factor, Ea: apparent activation energy, T: measuring temperature (°K), R: gas constant, and A: proportional constant).

The kneading status monitor index (P) is defined as a value of Ea calculated from either of the above equations.

Noticing that "reference kneading status monitor index (P)" used in this practical example corresponds to "target kneading status monitor index (P)" used in the second evaluation method of the invention, and also corresponds to the target kneading status monitor index (M0) used in the second evaluation method of the invention.

(3) Filler dispersion index (N) : After measuring strain dependency of a dynamic elastic modulus of an even thickness cross-linkable rubber sheet which is formed from an uncured rubber composition (the same formulation as the uncured rubber composition described in (1)) obtained by kneading at least an ethylene-$\alpha$-olefin based copolymer rubber and a reinforcing filler with a closed type mixer, and then further kneading the resultant kneaded material with a vulcanizing agent or a cross-linking agent, and if necessary, with a vulcanization accelerator or a cross-linking aid by using an 8-inch open roll mill, then a filler dispersion index (N) is calculated as a percentage of ( (E*(b)/E*(a)) x 100) (more precisely (|E*(b)|/|E*(a)|) x 100), where E*(a) is a dynamic elastic modulus (more precisely a complex modulus) at a given strain in a zone where a dynamic elastic modulus (more precisely a complex modulus) had little change against a strain change, and E*(b) is a dynamic elastic modulus (more precisely a complex modulus) at a given strain in a zone where a dynamic elastic modulus (more precisely a complex modulus) had a large change against a strain change.

(4) Kneading status monitor index (M) : Temperature dependency of a complex viscosity coefficient of an uncured rubber composition (the same formulation as the uncured rubber composition described in (2)), which is obtained by kneading at least an ethylene-$\alpha$-olefin based copolymer rubber with a reinforcing filler in a closed type mixer while a share stress is applied or both a share stress and heat are applied without any vulcanizing agent, any cross-linking agent, any vulcanization accelerator, nor any cross-linking aid, is shown in the following equation;

$$\eta^* = A \exp(-Ea/RT)$$

(where $\eta$ *: complex viscosity coefficient, Ea: apparent activation energy, T: measuring temperature (°K), R: gas constant, and A: proportional constant), or

$$a_T = A \exp(-Ea / RT)$$

(where $a_T$: shift factor, Ea: apparent activation energy, T: measuring temperature (°K), R: gas constant, and A: proportional constant).

The kneading status monitor index (M) is defined as a value of Ea calculated from either of the above equations.

As the reinforcing filler described above, a carbon black is preferably used.

The manufacturing methods for a rubber composition of the present invention, for example, the manufacturing method for an ethylene-α-olefin based copolymer rubber composition for cross-linking described above can provide an ethylene- α -olefin based copolymer rubber composition for cross-linking with good filler dispersion and a stable kneading status.

The ethylene-α-olefin based copolymer rubber composition for cross-linking in which at least ethylene-α-olefin based copolymer rubber and a reinforcing filler, specifically 30 parts by weight or more of the reinforcing filler per 100 parts by weight of the ethylene-α-olefin based copolymer rubber are kneaded by a closed type mixer and compounded with a vulcanizing agent or a cross-linking agent, and if necessary, a vulcanization accelerator or a cross-linking aid is characterized in that a ratio (N/R) of a filler dispersion index (N) to a filler dispersion index (R) obtained by an analysis with the method described above falls in the range of from 1 to 0.8 and/or a ratio (M/P) of a kneading status monitor index (M) to a target kneading status monitor index (P) obtained by an analysis with the method described above falls in the range of from 1 to 0.85.

As the reinforcing filler described above, a carbon black is preferably used.

This ethylene-α-olefin based copolymer rubber composition for cross-linking has a good filler dispersion and a very stable kneading status.

By adopting the ratio (N/R) between filler dispersion indexes described above and the ratio (M/P) between kneading status monitor indexes described above as objective evaluation indexes for filler dispersion and a kneading status respectively, which are newly found by the present inventors though, it becomes possible for the first time to easily set kneading conditions of a closed type mixer according to a shear condition and seasonally variable temperature and humidity. As a result, it becomes possible to manufacture stably an ethylene-α-olefin based copolymer rubber composition for cross-linking which has good filler dispersion, better processability for extrusion and injection molding, and capable of molding a product with better physical properties even by using a closed type mixer as a kneading machine.

## BRIEF DESCRIPTION OF THE DRAWINGS

Fig. 1 is a graph showing a strain dependency of a dynamic elastic modulus to explain a measurement method of a filler dispersion index.

Fig. 2 is a graph showing a relation between a complex viscosity coefficient ($\eta$*) and a frequency to explain a determination of an activation energy from a melt viscosity.

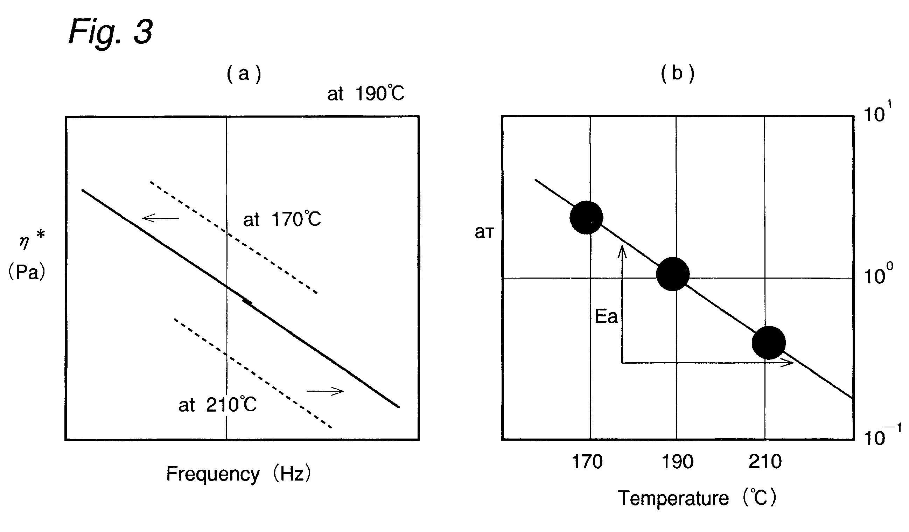

Fig. 3 (a) is a graph showing a relation between a complex viscosity coefficient ($\eta$*) and a frequency to explain a determination of an activation energy from a melt viscosity and (b) is a graph showing a relation between a shift factor ($a_T$) and a temperature.

## BEST MODE FOR CARRYING OUT THE INVENTION

The evaluation methods for kneading status of a rubber composition and the manufacturing methods for a rubber composition of the present invention will be specifically explained hereinafter.

At first, the rubber composition (I) to be used at the evaluation methods for kneading status of a rubber composition and the manufacturing methods for a rubber composition of the present invention will be described below.

## Rubber composition (I)

As the rubber (A) which is one component of the rubber composition (I) defined in the present invention, a natural rubber (NR) or a synthetic rubber can be used.

As the synthetic rubbers, specifically, an ethylene-α-olefin based copolymer rubber such as isoprene rubber (IR), styrene-butadiene rubber (SBR), butadiene rubber (BR), chloroprene rubber (CR), acrylonitrile-butadiene rubber (NBR), butyl rubber (IIR), ethylene-propylene rubber (EPR) and so on, an ethylene-α-olefin (non) -conjugate polyene copolymer rubber such as ethylene-propylene-diene rubber (EPDM) and so on, fluororubber and epichlorohydrin and so on can be exemplified.

As the filler (B) which is one component of the rubber composition (I) defined in the present invention, conventionally known reinforcing fillers or conventionally known non-reinforcing fillers can be used.

In addition, for the rubber composition (I) described above, if necessary, conventionally known additives such as a vulcanizing agent, a cross-linking agent, a vulcanization accelerator, a cross-linking aid, a softener, a heatproof stabilizer, a weatherproof stabilizer, an antistatic agent, a lubricant, a processing aid, and an anti pseudo-gel agent can be used within some amount not to cause a loss of an essential purpose of the present invention.

Next, the first evaluation method for kneading status of a rubber composition andthe first manufacturing method for a rubber composition of the present invention will be described.

**First evaluation method for kneading status of rubber composition**

The first evaluation method for kneading status of a rubber composition of the present invention is an evaluation method which is applied to the rubber composition (I) containing at least a rubber (A) and a filler (B), and comprises a complex modulus measurement step (1), a filler dispersion index calculation step (2), and a comparison step (3), which will be described below.

Technological concept of the evaluation method is explained below. A complex modulus $E^*$ of the rubber composition (I) containing at least a rubber (A) and a filler (B) changes (decreases) according to an increase of strain $\varepsilon$. Such the change is explained by that a destroy of aggregation and bonding of the filler (B) in the rubber composition (I) is promoted along with an increase of applied strain $\varepsilon$ and results in a decrease of complex modulus $E^*$.

Therefore, the rubber composition (I) in good filler dispersion must originally have a smaller change rate of complex modulus $E^*$ because aggregation and bonding of the filler (B) have been broken already. In other words, it can be said that the rubber composition (I) with a smaller change rate of complex modulus $E^*$ against a strain $\varepsilon$ is a better composition in filler dispersion, in contrast, the rubber composition (I) with a bigger change rate of complex modulus $E^*$ against a strain $\varepsilon$ is a worse composition in filler dispersion.

This change rate of complex modulus $E^*$ against a strain $\varepsilon$ can be evaluated in a ratio ($E^*(b)/E^*(a)$) between complex elastic moduli ($E^*(a)$ and $E^*(b)$) measured at two different strains ($\varepsilon a$ and $\varepsilon b$). Further, it can be simply evaluated by a ratio between a real part $E'(a)$ of $E^*(a)$ and a real part $E'(b)$ of $E^*(b)$.

**Complex modulus measurement step (1)**

In the complex modulus measurement step (1), a complex modulus $E^*a$ at a given strain $\varepsilon a$ and a complex modulus elastic $E^*b$ at a given strain $\varepsilon b$ which differs from the strain $\varepsilon a$ are measured for the rubber composition (I).

The complex modulus can be measured by a several kind of viscoelasticity measurement instrument. For instance, RDS or RSA II by Rheometric and RPA-2000 by Alpha Technologies can be mentioned as a representative of viscoelasticity measurement instruments but are not exclusive. In short, any type of viscoelasticity measurement instrument is acceptable as long as that it can measure a complex modulus at different strains with some degree of accuracy and stability.

The principle of a measurement for complex modulus is described in detail at, for example, page 39 through 42 of "Basics for Rubber Technologies" published by Corporation Aggregate Japan Rubber Association. As long as a complex modulus can be measured according to the principle, any type of measurement instrument is applicable for the present invention.

The measurement for complex modulus can be implemented on both a vulcanized rubber (preferably in rubber sheet) and an unvulcanized rubber (preferably in rubber sheet) . However, in case of the measurement for complex modulus of unvulcanized rubber, it is preferable to use a sample of unvulcanized rubber compound which does not contain any vulcanizing agent and so on to avoid a progress of vulcanization during a measurement.

Regarding the complex modulus, not only a complex modulus $E^*$ obtained by a measurement in a direction of Young's modulus (in tensile direction), but also a complex modulus $G^*$ obtained by a measurement in torsion direction can be applied to the present invention.

Specific examples of these measurement methods in each direction are described below.

(1) Measurement of complex modulus $E^*$ in the direction of Young's modulus (in tensile direction)

A sample of vulcanized rubber sheet in 1mm thickness which is punched out and sized in 10mm x 30mm is

8

attached to a sample holder. In this procedure, a sample of vulcanized rubber sheet should be attached to the holder avoiding deflection of a sample. By using a viscoelasticity measurement instrument like the RSA of Rheometric, a complex modulus E* is measured while an applied strain is varied from 0.01 to 2.2%.

In addition, as a similar variable, the strain dependency of E' (dynamic elastic modulus) can be used.

(2) Measurement of complex modulus G* in torsion direction

Basing on strain dependency of G*, a complex modulus G* can be measured by applying a strain in torsion direction onto a vulcanized rubber sheet in 2mm thickness, and also G' (dynamic elastic modulus) can be used as a similar variable.

In addition, the measurement of complex modulus G* can be applied on not only a vulcanized rubber sheet but also unvulcanized rubber compound and can be evaluated as described above.

The measurement for a complex modulus G* can be proceeded on an unvul canized rubber compound which contains a vulcanization agent and a vulcanization accelerator, and however, the measurement of an unvulcanized rubber compound requires attention on a measurement temperature to prevent a progress of vulcanization during a measurement.

### Filler dispersion index calculation step (2)

In the filler dispersion index calculation step (2), a filler dispersion index (N) of the rubber composition (I) is calculated according to the following equation from complex elastic moduli E*(a) and E*(b) obtained at the complex modulus measurement step (1).

$$\text{Filler dispersion index (N)} = |E^*(a)| \,/\, |E^*(b)|$$

### Comparison step (3)

In the comparison step (3), a predetermined target filler dispersion index (R) is compared with the filler dispersion index (N) calculated in the filler dispersion index calculation step (2).

The target filler dispersion index (R) is generally a complete target filler dispersion index (NO) which is obtained through the complex modulus measurement step (1) and the filler dispersion index calculation step (2) after a rubber composition having the same formulation as that of the rubber composition (I) is substantially kneaded to achieve a practically complete dispersion. The target filler dispersion index (R) can be obtained from either theoretically calculated values by a method like a computer simulation or the measurement values described above.

The term "practically complete dispersion" described above means a kneading status in which a filler has substantially dispersed in the composition described above and filler dispersion will not improve anymore even if further kneading energy is applied. A kneading energy can be measured, for example, as a power consumed by a mixer. A degree of filler dispersion can be indirectly estimated, for example, by measuring physical properties (a hardness, a tensile strength, a tensile, and so on) after vulcanizing the composition described above. Therefore, the term "practically complete dispersion" can be said as, for example, a saturated state in changes of various physical properties of a vulcanized rubber while power consumption by a mixer is increasing. The practically complete dispersion state exits for each rubber composition which has a different formulation of ingredients.

The practically complete dispersion is preferably achieved by kneading with an open roll mill. The kneading with an open roll mill can provide an almost ideal kneading status, so that a composition kneaded with an open roll mill can be used to measure a complete target filler dispersion index (NO) assuming that a composition kneaded with an open roll mill is in a practically complete dispersion state.

The alternate simple evaluation method of the first evaluation method for kneading status of a rubber composition of the present invention is similar to the method described above.

### First manufacturing method for rubber composition

In the first manufacturing method for a rubber composition of the present invention, the first evaluation method for kneading status of a rubber composition of the present invention (including an alternate simple evaluation method) is carried out. Through this operation, a filler dispersion state of the rubber composition (I) can be objectively evaluated.

This manufacturing method, in general, further comprises a feedback step (4) to control kneading conditions of the rubber composition (I) according to the result from the comparison step (3) described above and by means of adjusting a value of filler dispersion index (N)/target filler dispersion index (R) to be a certain numeric range.

The numeric range of the value of filler dispersion index (N) / target filler dispersion index (R) (where $|E^*(a)| \leq |E^*(b)|$) is preferably 0.8 to 1.0.

Regarding kneading conditions of the rubber composition (I), a kneading temperature, a kneading time, a sheer rate, a floating weight pressure, the number and/or timing of floating weight up-down movement, a mixer fill factor, a wing density of mixer, a clearance between a wing and a casing of mixer, a clearance between rotors, and so on can be listed up.

The second evaluation method for kneading status of a rubber composition and the second manufacturing method for a rubber composition of the present invention will be described below.

**Second evaluation method for kneading status of rubber composition**

The second evaluation method for kneading status of a rubber composition of the present invention is an evaluation method applied to an uncured rubber of the rubber composition (I) containing at least a rubber (A) and a filler (B), and comprises a complex viscosity coefficient measurement step (5), a kneading status monitor index calculation step (6), and a comparison step (7), which will be described below.

Technological concept of this evaluation method is explained below. As is clear by the following equation, the M used in the equation shows temperature dependency of a complex viscosity coefficient $\eta *$.

$$| \eta *(T)| = A \exp (-M / RT)$$

In this equation, A is a proportional constant, R is the gas constant, and T is a measuring temperature (°K). The temperature dependency of a complex viscosity coefficient $\eta *$ becomes weaker according to increase of M value, in contrast, the temperature dependency of a complex viscosity coefficient $\eta *$ becomes stronger according to decrease of M value.

An influence of pseudo-gel formation and deformation in the rubber composition (I) becomes stronger according to an increase of temperature dependency of a complex viscosity coefficient $\eta *$. The pseudo-gel formation and deformation scarcely occurs in the rubber composition (I) which is in a good kneading status, and hence the temperature dependency of a complex viscosity coefficient $\eta *$ becomes minimum while M value becomes maximum. In other words, it can be said that the rubber composition (I) with a large M value is a rubber composition which is in a good kneading status, and the rubber composition (I) with a small M value is a rubber composition which is in a bad kneading status.

Consequently, the kneading status of the rubber composition (I) can be objectively evaluated by measuring a complex viscosity coefficient $\eta *$ at two or more different temperatures and by calculating M value from the Arrhenius plots.

For a reference, there are two calculation methods for M value. The M value can be obtained from the equation (1) or the equation (2) shown below. The -Ea in these equations corresponds to M value.

$$| \eta *| = A \exp (-Ea/RT) \tag{1}$$

In this equation (1), $\eta *$ is a complex viscosity coefficient, Ea is an apparent activation energy, T is a measuring temperature (°K), R is the gas constant, and A is a proportional constant.

$$a_T = A \exp (-Ea / RT) \tag{2}$$

In this equation (2), $a_T$ is a shift factor, Ea is an apparent activation energy, T is a measuring temperature (°K), R is the gas constant, A is a proportional constant.

**Complex viscosity coefficient measurement step (5)**

In the complex viscosity coefficient measurement step (5), a complex viscosity coefficient $\eta *$ of the rubber composition (I) is measured under at least two different temperatures.

**Kneading status monitor index calculation step (6)**

The complex viscosity coefficient can be measured by a several kind of viscoelasticity measurement instrument. For instance, RDS or RSA II by Rheometric and RPA-2000 by Alpha Technologies can be mentioned as a representative of viscoelasticity measurement instruments but are not exclusive. In short, any type of viscoelasticity measurement instrument is acceptable as long as it can measure a complex viscosity coefficient with some degree of accuracy and stability.

The principle of a measurement for complex viscosity coefficient is described in detail at, for example, page 39 through 42 of "Basics for Rubber Technologies" published by Corporation Aggregate Japan Rubber Association. As long as a complex viscosity coefficient can be measured according to the principle, any type of measurement instrument is applicable for the present invention.

The measurement for a complex viscosity coefficient can be implemented on an unvulcanized rubber compound. In this case, as a test sample, it is preferable to use an unvulcanized rubber compound which does not contain any vulcanizing agent and so on, so that no vulcanization reaction can progress during a measurement. In case of using a sample of unvulcanized rubber compound which contains a vulcanizing agent and so on, it is preferable to keep a measurement temperature below the temperature at which vulcanization starts, so that no vulcanization reaction can progress.

The complex viscosity coefficient can be measured on an unvulcanized rubber but not on a vulcanized rubber. Though an unvulcanized rubber compound may contain a vulcanization agent and a vulcanizing aid, it is necessary to have a proper measurement temperature setting, so that no vulcanization starts during a measurement. Therefore, as a test sample, it is preferable to use an unvulcanized rubber compound which does not contain any vulcanization agent and aid. Therefore, it is preferable to use an unvulcanized rubber compound sampled immediately after kneading by a Banbury mixer and so on.

The complex viscosity coefficient can be measured with RDS of Rheometics or with RPA-2000 of Alpha Technologies. After measuring a complex elastic coefficient $\eta^*$ or a shift factor $a_T$ at each measurement temperature, an activation energy (Ea) is calculated from the equation (1) or (2) shown above. As the measuring temperature, two conditions are theoretically enough, but three or more conditions are preferred from the viewpoint of accuracy.

RPA-2000 of Alpha Technologies can provide a complex viscosity coefficient measurement in a simple manner, in which no sample preparation such as making a sheet of unvulcanized rubber compound is necessary except weighing a compound not less than a weight corresponding to its cavity volume.

In the kneading status monitor index calculation step (6), on a bases of temperature dependency of complex viscosity coefficient $\eta^*$ which is obtained at the complex viscosity coefficient measurement step (5), a kneading status monitor index (M) of the rubber composition (I) can be calculated according to the following equation;

$$|\eta^*(T)| = A \exp(-M/RT)$$

(where $\eta^*$: complex viscosity coefficient, A: proportional constant, R: gas constant and T: measuring temperature ($^\circ$K)).

### Comparison step (7)

In the comparison step (7), a predetermined target kneading status monitor index (P) is compared with the kneading status monitor index (M) calculated in the kneading status monitor index calculation step (6).

The target kneading status monitor index (P) is generally a complete target kneading status monitor index (M0) which is obtained through the complex viscosity coefficient measurement step (5) and the kneading status monitor index calculation step (6) after a rubber composition having the same formulation as that of the rubber composition (I) is substantially kneaded to achieve a practically complete dispersion. The target kneading status monitor index (P) can be obtained from either theoretically calculated values by a method like a computer simulation or the measurement values described above.

The term "practically complete dispersion" described above means a kneading status in which a filler has substantially dispersed in the rubber composition described above and filler dispersion will not improve anymore even if further kneading energy is applied. A kneading energy can be measured, for example, as a power consumed by mixer. A degree of filler dispersion can be indirectly estimated, for example, by measuring physical properties (a hardness, a tensile strength, a tensile, and so on) after vulcanizing the rubber composition described above. Therefore, the term "practically complete dispersion" can be said as, for example, a saturated state in changes of various physical properties of a vulcanized rubber while power consumption by a mixer is increasing. The practically complete dispersion state exits for each rubber composition which has a different formulation of ingredients.

The practically complete dispersion is preferably achieved by kneading with an open roll mill. The kneading with an open roll mill can provide an almost ideal kneading status, so that a composition kneaded with an open roll mill can be used to measure a complete target kneading status monitor index (M0) assuming that a composition kneaded with an open roll mill is in a practically complete dispersion state.

**Second manufacturing method for rubber composition**

This second manufacturing method for a rubber composition of the present invention is characterized by utilizing the second evaluation method for kneading status of a rubber composition of the present invention as described above. Through this operation, a kneading status of the rubber composition (I) can be objectively evaluated.

This second manufacturing method, in general, further comprises a feedback step (8) to control a kneading condition for the rubber composition (I) by means of adjusting a value of kneading status monitor index (M)/target kneading status monitor index (P) to be a certain numeric range according to a result from the comparison step (7) described above.

The numeric range of the value of kneading status monitor index (M) / target kneading status monitor index (P) is preferably 0.85 to 1.0.

Regarding kneading conditions of the rubber composition (I), a kneading temperature, a kneading time, a sheer rate, a floating weight pressure, the number and/or timing of floating weight up-down movement, a mixer fill factor, a wing density of mixer, a clearance between a wing and a casing of mixer, a clearance between rotors, and so on can be listed up.

The first manufacturing method for a rubber composition of the present invention may contain the steps ( (5) to (7), and (8)) of the second manufacturing method for a rubber composition of the present invention. On the contrary, the second manufacturing method for a rubber composition of the present invention may contain the steps ( (1) to (3), and (4)) in the first manufacturing method for a rubber composition of the present invention. In other words, both the first and the second kneading status evaluation method for a rubber composition described above can be applied at a same time to the manufacturing method of the present invention.

The present invention can be implemented in various applications according to a kind, nature, and usage of a rubber composition, and however, for instance, the following manufacturing method for an ethylene-$\alpha$-olefin based copolymer rubber composition for cross-linking can be exemplified.

In the manufacturing method for an ethylene-$\alpha$-olefin-based copolymer rubber composition for cross-linking, a rubber composition for cross-linking is manufactured by kneading in a closed type mixer at least ethylene-$\alpha$-olefin based copolymer rubber and a reinforcing filler; specifically 30 parts by weight or more of a carbon black per 100 parts by weight of the ethylene-$\alpha$-olefin based copolymer rubber, and compounding with a vulcanizing agent or a cross-linking agent, if necessary, a vulcanization accelerator or a cross-linking aid, and additives commonly used as a softener like olefin rubber additives.

There is no specific limitation regarding a kind of ethylene-$\alpha$-olefin based copolymer rubber used in the present invention, so that any conventionally known type of ethylene-$\alpha$-olefin based copolymer rubber can be used. For example, an ethylene-$\alpha$-olefin copolymer rubber such as EPR and an ethylene-$\alpha$-olefin non-conjugated polyene copolymer rubber such as EPDM can be exemplified.

As the reinforcing filler used in the present invention, conventionally known reinforcing fillers can be used, specifically, carbonblack, silicicacidanhydride, silicicacid hydride, potassium silicate, aluminum silicate, clay, talc, and calcium carbonate are exemplified. Of these, carbon black is preferably used.

The dosage of the reinforcing filler such as a carbon black varies depending on a usage of ethylene-$\alpha$-olefin based copolymer rubber composition for cross-linking, and however, 30 parts by weight or more, generally 30 to 300 parts by weight, preferably 60 to 300 parts by weight, and more preferably 100 to 300 parts by weight of a reinforcing filler is used based on 100 parts by weight of the ethylene-$\alpha$-olefin based copolymer rubber.

As the vulcanizing agents used in the present invention, conventionally known vulcanizing agents such as sulfur and sulfur compound can be exemplified.

In the present invention, it is preferable to use a vulcanization accelerator together with these vulcanizing agents. The vulcanization accelerator is not specifically limited as long as the vulcanization accelerator is conventionally known.

Further, as the cross-linking agents used in the present invention, an organic peroxide and so on is exemplified. The organic peroxide is not specifically limited as long as the organic peroxide has been conventionally used for cross-linking of EPR and EPRM.

In the present invention, it is preferable to use a cross-linking aid together with an organic peroxide. The cross-linking aid is not specifically limited as long as the cross-linking aid is conventionally known.

In addition, in the manufacturing method for ethylene-$\alpha$-olefin based copolymer rubber composition for cross-linking of the present invention, if necessary, conventionally known additives such as a softener, a heatproof stabilizer, a weatherproof stabilizer, an antistatic agent, a lubricant, a processing aid, and a pseudo-gel preventing agent can be used with a proper amount in which an inherent purpose of the present invention will not be lost.

As the closed type mixer, so-called Banbury mixer, Kneader, Intermix, and Werner can be specifically exemplified.

In the manufacturing method for ethylene-$\alpha$-olefin based copolymer rubber composition for cross-linking of the present invention, at first, analyses is carried out in advance according to the following procedures to obtain a filler dispersion index (R) and/or a kneading status monitor index (P) .

(1) Reference filler dispersion index (R): After measuring strain dependency of a dynamic elastic modulus (more precisely complexmodulus) of an even thickness cross-linkable rubber sheet which is formed from an unvulcanized rubber composition obtained by kneading at least an ethylene- $\alpha$-olefin based copolymer rubber, a reinforcing filler, a vulcanizing agent or a cross-linking agent, and if necessary, a vulcanization accelerator or a cross-linking aid at a temperature of 100°C or lower by using an 8-inch open roll mill, then a reference filler dispersion index (R) is calculated as a percentage of ( (E* (b) /E* (a)) x 100) (more precisely ($|E^*$ (b) $|$ / $|E^*$ (a)$|$) x100) where E* (a) is a dynamic elastic modulus (more precisely a complex modulus) at a given strain in a zone where a dynamic elastic modulus (more precisely a complex modulus) had little change against a strain change, and E*(b) is a dynamic elastic modulus (more precisely a complex modulus) at a given strain in a zone where a dynamic elastic modulus (more precisely a complex modulus) had a large change against a strain change.

The strain dependency of dynamic elastic modulus of an even thickness cross-linkable rubber sheet can be measured by using a viscoelasticity measurement instrument like RSA II of Rheometirics, but this is not exclusive. This measurement method will be described in detail in the section of application example.

The term "zone where a dynamic elastic modulus had little change against a strain change" described above means an area where a change rate of a dynamic elastic modulus becomes less than 3% among the above vulcanized rubber sheets (including cross-linked rubber sheets). (This definition will be effective hereafter.) Also, the term "zone where a dynamic elastic modulus had a large change against a strain change" described above means an area where a change rate of a dynamic elastic modulus becomes 3% or larger among the above vulcanized rubber sheets. (This definition will be effective hereafter.)

(2) Reference kneading status monitor index (P) : Temperature dependency of a complex viscosity coefficient of an unvulcanized rubber composition obtained by kneading at least an ethylene-$\alpha$-olefin based copolymer rubber with a reinforcing filler at a temperature of 100°C or lower by using an 8-inch open roll mill without any vulcanizing agent, any cross-linking agent, any vulcanization accelerator, noranycross-linkingaidis shown in the following equation;

$$\eta^* = A \exp (-Ea / RT)$$

(where $\eta^*$: complex viscosity coefficient, Ea: apparent activation energy, T:measuring temperature (°K), R: gas constant, and A: proportional constant); or

$$a_T = A \exp (-Ea / RT)$$

(where $a_T$: shift factor, Ea: apparent activation energy, T: measuring temperature (°K), R: gas constant, and A: proportional constant).

The reference kneading status monitor index (P) is defined as a value of Ea calculated from either of the above equations.

The kneading status monitor index (P) can be measured by using a viscoelasticity measurement instrument like RSA II of Rheometirics, but this is not exclusive. This measurement method will be described in mor detail in the section of application example.

Next, in the case of a production of ethylene-$\alpha$-olefin based copolymer rubber composition for cross-linking, at least an ethylene-$\alpha$-olefin based copolymer rubber and a reinforcing filler in the same compounding ratio as that described above, and if necessary, a vulcanizing agent or a cross-linker, a vulcanization accelerator or a cross-linking aid, and a generally used additive such as a softener for olefin-based rubber in the same formulation as that described above are kneaded by a closed type mixer, and then analyses is carried out according to the following procedures to obtain a filler dispersion index (N) and/or a kneading status monitor index (M).

(3) Filler dispersion index (N): After measuring strain dependency of a dynamic elastic modulus of a cross-linkable even thickness rubber sheet which is formed from an uncured rubber composition (the same formulation as the uncured rubber composition described in (1)) obtained by kneading at least an ethylene-$\alpha$-olefin based copolymer rubber and a reinforcing filler with a closed type mixer, and then kneading the resultant kneaded material with a vulcanizing agent or a cross-linking agent, and if necessary with a vulcanization accelerator or a cross-linking aid by using an 8-inch open roll mill, then a filler dispersion index (N) is calculated as a percentage of ( (E*(b) /E* (a) ) x 100) (more precisely ($|E^*(b)|$/$|E^*(a)|$) x 100) where E* (a) is a dynamic elasticmodulus (more precisely a complex modulus) at a given strain in a zone where a dynamic elastic modulus (more precisely a complex modulus) had little change against a strain change, and E* (b) is a dynamic elastic modulus (more precisely a complex modulus) at a given strain in a zone where a dynamic elastic modulus (more precisely a complex modulus) had a large change against a strain change.

The filler dispersion index (N) can be measured by using a viscoelasticity measurement instrument like RSA II of Rheometirics, but this is not exclusive. This measurement method will be described in detail in the section of application example.

(4) Kneading status monitor index (M) : Temperature dependency of a complex viscosity coefficient for an uncured rubber composition (the same formulation as the uncured rubber composition (2) described above), which is obtained by kneading at least an ethylene-α-olefin based copolymer rubber with a reinforcing filler in a closed type mixer while a share stress is applied or both a share stress and heat are applied without any vulcanizing agent, any cross-linking agent, any vulcanization accelerator, nor any cross-linking aid, is shown in the following equation;

$$\eta^* = A \exp(-Ea / RT)$$

(where $\eta^*$: complex viscosity coefficient, Ea: apparent activation energy, T: measuring temperature (°K), R: gas constant, and A: proportional constant), or

$$a_T = A \exp(Ea / RT)$$

(where $a_T$: shift factor, Ea: apparent activation energy, T: measuring temperature (°K), R: gas constant, and A: proportional constant).

The kneading status monitor index (M) is defined as a value of Ea calculated from either of the above equations.

The kneading status monitor index (M) can be measured by using a viscoelasticity measurement instrument like RSA II of Rheometirics, but this is not exclusive. This measurement method will be described in detail in the section of application example.

The kneading conditions with a closed type mixer are controlled such that a filler dispersion index (N) obtained by the method described above satisfies the following equation;

$$\text{Filler dispersion index (N) / Reference filler dispersion index (R) =}$$

$$1 \text{ to } 0.8,$$

and/or such that a kneading status monitor index (M) obtained by the method described above satisfies the following equation;

$$\text{Kneading status monitor index (M) / Reference kneading status}$$

$$\text{index (P)} = 1 \text{ to } 0.85.$$

When the value of filler dispersion index (N) / target filler dispersion index (R) is within from 1 to 0.8, it can be evaluated that the filler dispersion of a rubber composition kneaded by a closed type mixer is good.

When the value of kneading status monitor index (M)/target kneading status monitor index (P) is smaller than 0.85, there is a possibility of occurrence of pseudo-gel in a rubber composition kneaded by a closed type mixer. If such a pseudo-gel occurs, a die swell in an extrusion molding becomes small resulting in worse physical properties of a vulcanized (cross-linked) rubber. For a reference, this change can not be observed in Mooney viscosity value (ML (1+4) 100°C) which is normally used as a control index to monitor a kneading status.

The present inventors found that, in a compounding system like an ethylene-α-olefin based copolymer rubber compounded with a carbon black, the longer time a composition is kneaded, the more pseudo-gels appear on an interface between a polymer and a filler (carbon black), and the occurrence of pseudo-gels can be prevented by adding oxygen (air) which acts as a radical capturing effect at the place where pseudo-gels occur.

Oxygen can be supplied into a closed type mixer by moving a floating weight up and down. However, repeating this operation will result in lowering a pressure force on a rubber composition, making poor filler dispersion, prolonging a duration of kneading time, and decreasing a production speed of an objective ethylene-α-olefin based copolymer rubber composition for cross-linking.

In the manufacturing methods for an ethylene-α-olefin based copolymer rubber composition for cross-linking of the present invention, both a filler dispersion index by which filler dispersion can be objectively evaluated and a kneading status monitor index by which a kneading status can be objectively evaluated, which have been found by the present inventors, are adopted to control kneading conditions of a closed type mixer by means of adjusting a filler dispersion index (N) to satisfy the following equation;

Filler dispersion index (N) / target filler dispersion index (R) = 1 to 0.8

and/or by means of adjusting a kneading status monitor index (M) to satisfy the following equation;

Kneading status monitor index (M)/ target kneading status monitor index (P) =

1 to 0.85.

For example, by moving a floating weight mounted on a closed type mixer up and down, oxygen can be supplied into the closed type mixer, so that occurrence of pseudo-gels can be minimized, and as a result, it becomes possible to produce most economically an ethylene-$\alpha$-olefin based copolymer rubber composition for cross-linking which has better filler dispersion and a stable kneading status. The floating weight works as a weight for a closed part of the mixer, and its up-and-down movement is, in general, a performance to scrape off (clean up) compounding materials which are blown up on a top part of it.

If a formulation of a rubber composition to be evaluated is different from that of a rubber composition, with which a target filler dispersion index (R) and a target kneading status monitor index (P) of a rubber composition are defined in the present invention, these indexes cannot be compared respectively with a filler dispersion index (N) and a kneading status monitor index (M) of a rubber composition to be evaluated, and thus it is necessary to make a formulation of a rubber composition to be evaluated the same as that of the rubber composition defined in the above description. In other words, when a formulation of a rubber composition to be evaluated is changed, a target filler dispersion index (R) and a target kneading status monitor index (P) should be newly obtained from the rubber composition whose composition is changed.

After finding a target filler dispersion index (R) and/or a target kneading status monitor index (P) of a rubber composition obtained by an 8-inch open roll mill, which is believed in the present invention as that it will provide the best kneading status of a rubber composition, and by using these indexes as reference values, comparisons between a target filler dispersion index (R) and/or a target kneading status monitor index (P) with respectively a filler dispersion index (N) and/or a kneading status monitor index (M) of a rubber composition obtained by a closed type mixer are carried out to make it possible to know a shift from an ideal kneading status and to adjust conditions of a closed type mixer, specifically a compounding fill factor, a rotation speed, and a timing of up-and-down movement of a floating weight, so that an ideal kneading status can be achieved.

However, the kneading method with an 8-inch open roll mill can provide a better kneading status, but it is not suitable for a mass production of rubber composition.

Regarding the 8-inch open roll mill, pseudo-gels does not occur because oxygen is always supplied to a portion where a shear stress is added, a gap between rolls is small enough to produce a high shear stress, and it is possible to knead while controlling a material flow rate manually. Thus, it can provide excellent filler dispersion and a stable kneading status. A rubber composition kneaded by an 8-inch open roll mill can be adopted as a reference rubber composition in order to know a degree of filler dispersion and a kneading status of a rubber composition kneaded by a closed type mixer.

According to the manufacturing methods of an ethylene-$\alpha$-olefin based copolymer rubber composition for cross-linking of the present invention, it is possible to produce economically an ethylene-$\alpha$-olefin based copolymer rubber composition for cross-linking which has excellent filler dispersion and a stable kneading status. Specifically, an ethylene-$\alpha$-olefin based copolymer rubber composition with a reinforcing filler and so on can be kneaded without any occurrence of ribbon cracks, and also the obtained rubber composition has good processability of extrusion and injection molding, thus it can provide a mold product having good mechanical characteristics such as a tensile strength, a compression permanent strain performance, and so on.

The ethylene- $\alpha$-olefin based copolymer rubber composition for cross-linking of the present invention has good filler dispersion and a stable kneading status because the ratio (N/R) of a filler dispersion index (N) to a target filler dispersion index (R) described above is controlled to be within from 1 to 0.8, and/or the ratio (M/P) of a kneading status monitor index (M) to a target kneading status monitor index (P) described above is controlled to be within from 1 to 0.85.

**EFFECT OF THE INVENTION**

According to the evaluation methods for kneading status of a rubber composition of the present invention, a kneading status of a rubber composition containing at least a rubber and a filler can be objectively evaluated.

Further, according to the manufacturing methods for a rubber composition of the present invention, a rubber composition having good filler dispersion and a stable kneading status can be provided because objective evaluation methods for kneading status of a rubber composition are adopted.

**Examples**

The present invention will be explained with Examples below, but the present invention will not receive any restriction from these application examples.

For a reference, a tensile strength ($T_B$), a tensile elongation (Eb), and a compression set (Cs) used in Examples and Comparative Examples were determined in accordance with JIS K6253.

A filler dispersion index and a kneading status monitor index in Examples and Comparative Examples were determined under the following conditions respectively.

**(1) Filler dispersion index**

| Viscoelasticity Measurement Instrument RSA II made by Rheometric Scientific, Inc.; | |
|---|---|
| Frequency range | 0.0016 to 16 Hz |
| Amplification range | ± 0.5 mm |
| Strain resolution | ± 0.05 µm |
| Maximum load | 9.81 N |
| Phase angle resolution | ± 0.1 degree |
| Measurement sensitivity | 1 g |
| Temperature gradient rate | 0.1 degrees to 50 degrees/min |
| Condition of measurement; | |
| Initial load | 50 g (to remove flexure from a narrow rectangle specimen attached) |
| Strain | 0.01 to 2% |
| Frequency | 10 Hz |
| Measuring temperature 25°C (with a temperature control) | |
| Measurement | strain dependency of dynamic elastic modulus (dynamic Young's modulus) (more precisely, a complex modulus (E*)) |

A narrow rectangle specimen for measurement of a dynamic elastic modulus was attached to the viscoelasticity measurement instrument described above, and after confirming that there is no flexure of the narrow rectangle specimen attached, strain dependency of a dynamic elastic modulus (more precisely, a complex modulus (E*)) was measured.

**(2) Kneading status monitor index**

| Viscoelasticity Measurement Instrument RDS II made by Rheometric Scientific, Inc.; | |
|---|---|
| Converter | |
| Torque range | 2,000 mg-cm |
| Drift | 0.1% at full scale of time |

(continued)

| Viscoelasticity Measurement Instrument RDS II made by Rheometric Scientific, Inc.; | |
| --- | --- |
| Converter | |
| Condition of measurement | |
| Initial load<br>Strain<br>Frequency<br>Measuring temperature 210°C, 190°C, and 170°C (with a temperature control) | 0 g<br>1%<br>10 Hz |
| Measurement | Ea (activation energy in kJ/mol) is calculated from the temperature dependency (Fig.3) of a shift factor $a_T$ at 190°C induced from the temperature dependency of a complex viscosity coefficient ($\eta^*$.) |

< Calculation of Activation Energy >

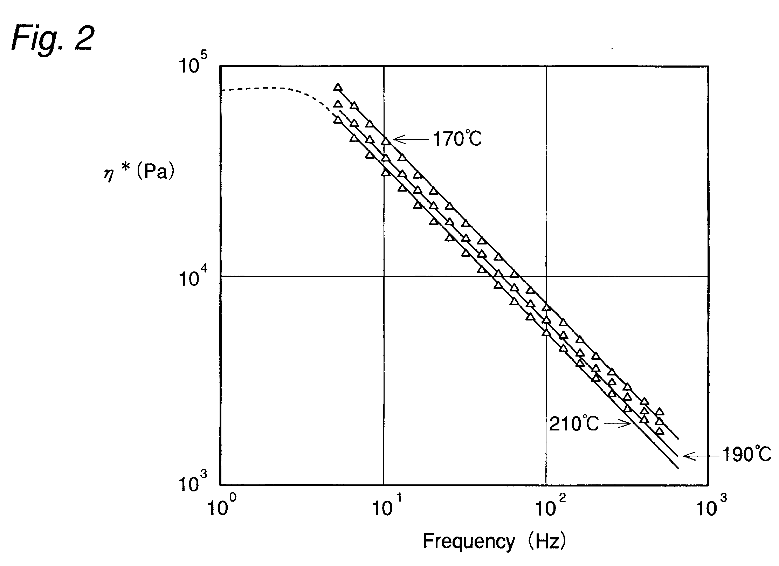

(1) As shown in Fig. 2, regarding a kneaded rubber compound, obtain a relation between a complex viscosity coefficient ($\eta^*$) and a frequency at given temperatures by measurements.
(2) On a basis of WLF shift, calculate a shift factor ($a_T$) from the following equation seeking for a relation between a complex viscosity coefficient ($\eta^*$) and a frequency at 190°C, and then calculate an activation energy (kJ/mol; Ea) from temperature dependency of the shift factor ($a_T$) ($a_T = A \exp (-Ea/R(T-Tref.))$).

WLF equation;

$$\eta_T = a_T\, \eta_{T0}$$

where a viscosity at the temperature T is designated $\eta_T$ and a viscosity at a reference temperature T0 is designated $\eta_{T0}$.

Examples 1 to 6

Target kneading status monitor index (P)

100 parts by weight of an ethylene-propylene-5-vinyl-2-norbornene copolymer rubber (molar ratio of ethylene/propylene = 70/30, iodine value = 20) as an ethylene-$\alpha$-olefin based copolymer rubber, 165 parts by weight of a carbon black (trade name: Asahi 60HG by Asahi Carbon K.K.), and 70 parts by weight of a softener (trade name: PW-380) were kneaded at 60 °C by using an 8-inch open roll mill to obtain an unvulcanized rubber composition which does not contain any vulcanizing agent and any vulcanization accelerator.
Then, about 20 g of the unvulcanized rubber composition was sampled, pressed by a 50-ton press machine at 160°C for 6 minutes, followed by 2 minutes press with residual heat, and cooled down with water for 5 minutes. Thus, an unvulcanized rubber sheet in 2 mm thickness with a square shape of 10cm x 10cm was obtained.
This unvulcanized rubber sheet was punched out into a couple of disc specimen in 25 mm diameter for a complex viscosity coefficient measurement. Regarding this specimen, a complex viscosity coefficient ($\eta^*$) was measured by using parallel plates of a viscoelasticity measurement instrument RDS II by Rheometric Scientific, Inc. under the conditions described above.
In detail, the specimen was heated up to 210°C and kept for 6 minutes so that an inside temperature of a chamber becomes stable at 210°C, thereafter, complex viscosity coefficients ($\eta^*$) were measured at 210°C, 190°C, and 170°C, and then a shift factor ($a_T$) was calculated from the equation described above. More specifically, in the case of a sequential measurement of complex viscosity coefficients ($\eta^*$) at each temperatures of 210°C, 190°C, and 170°C, at first, a complex viscosity coefficient at 210°C was measured after the specimen was heated up to 210°C and kept for 6 minutes so that an inside temperature of a chamber becomes stable at 210°C, then a temperature was changed at a rate of -5°C/min from 210°C to 190°C. After the specimen was kept for 6 minutes so that an inside temperature of a chamber becomes stable at 190°C, a complex viscosity coefficient ($\eta$ *) at 190°C was measured, and then a complex viscosity coefficient ($\eta$*) at 170°C was measured after reaching 170°C in the same manner as described above. Subsequently, on the basis of relation between a shift factor ($a_T$) and a measurement temperature (T), an apparent activation energy value (Ea) (kJ/mol), that is, a target kneading status monitor index (P) was

**Target filler dispersion index (R)**

An amount of 300gr of the unvulcanized rubber composition described above which does not contain any vulcanizing agent and any vulcanization accelerator was wound around an 8-inch open roll mill, and 1.5 phr of Sulfur as a vulcanizing agent, 0.5 phr of NOCCELER M (trade name, by Oouchi Sinko Kagaku Kogyo K.K.) and 1.0 phr of NOCCELER TT (trade name, by Oouchi Sinko Kagaku Kogyo K.K.) as vulcanization accelerators were added, and then they were kneaded at 70°C to obtain an unvulcanized rubber composition.

Then, the unvulcanized rubber composition was vulcanized at 160°C for 8 minutes under a pressure supplied by a 50-ton press machine to make a vulcanized rubber sheet in 1 mm thickness. This vulcanized rubber sheet was punched out into a couple of narrow rectangular specimen with 10 mm in width and 30 mm in length.

Regarding the narrow rectangular sample, the strain dependency of a dynamic elastic modulus was measured in the same manner as describe before by using the viscoelasticity measurement instrument RSA II made by Rheometric Scientific, Inc. described above.

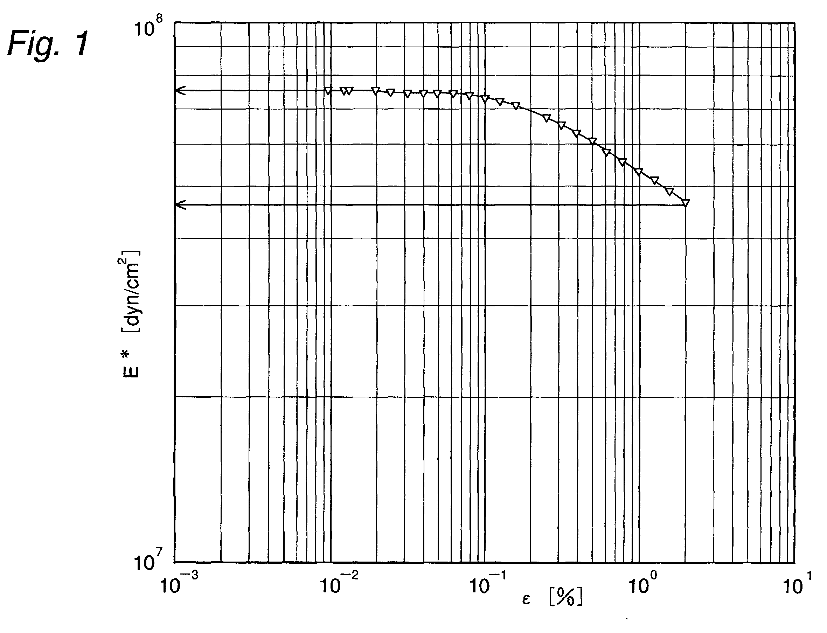

Then, the strain dependency of a dynamic elastic modulus was plotted on a graph. For example, as shown in Fig.1, after finding a dynamic elastic modulus (more precisely a complex modulus) E*(a) at 0.01% of strain ($\varepsilon$) which was in a zone where a dynamic elastic modulus (more precisely a complex modulus) (E*) had little change, and a dynamic elastic modulus (more precisely a complex modulus) E*(b) at 2% of strain ($\varepsilon$) which was in a zone where a dynamic elastic modulus (more precisely a complex modulus) had a large change, a target filler dispersion index (R) was calculated from the following equation.

$$R(\%)=(E^*(b)/E^*(a))\times100$$

(More precisely, R (%) = ($|E^*(b)|$ / $|E^*(a)|$) x 100)

**Kneading status monitor index (M)**

100 parts by weight of an ethylene-propylene-5-vinyl-2-norbornene copolymer rubber (molar ratio of ethylene/propylene = 70/30, iodine value = 20) as an ethylene-$\alpha$-olefin based copolymer rubber, 30 parts by weight of a carbon black (trade name: Asahi 60HG by Asahi Carbon K.K.), and 70 parts by weight of a softener (trade name: PW-380) were kneaded at 50°C of an initial kneading temperature by using a Kobe Seikosyo 1.7 liter BB2 type Banbury which is a closed type mixer (Banbury mixer, hereafter). Temperatures of dumping-out at each time of 50, 110, 240, and 360 seconds were 132, 145, and 175°C, respectively. Under these kneading conditions, an unvulcanized rubber composition not containing any vulcanizing agent and any vulcanization accelerator was obtained.

The kneading method with the closed type mixer described above, which was carried out in Example 1, Example 2, Example 3, and Example 5, in accordance with the kneading method (A1 method) specified at JIS K6299, and their kneading durations of time were 110 sec, 50 sec, 240 sec, and 360 sec, respectively.

The kneading method with the closed type mixer described above, which was carried out in Example 4 and Example 6, in accordance with the kneading method (A2 method) specified in JIS K6299, and a floating weight which was used for cleaning was moved up and down twice during the kneading. Their kneading durations of time were 240 sec and 360 sec, respectively.

Then, about 20 g of the unvulcanized rubber composition not containing any vulcanizing agent and any vulcanization accelerator described above was sampled, pressed by a 50-ton press machine at 160°C for 6 minutes, followed by 2 minutes press with residual heat, and cooled down with water for 5 minutes. Thus, an unvulcanized rubber sheet in 2 mm thickness with a square shape of 10cm x 10cm was obtained.

This unvulcanized rubber sheet was punched out into a couple of disc specimen in 25 mm diameter for a complex viscosity coefficient measurement. Regarding this specimen, a complex viscosity coefficient ($\eta$ *) was measured by using parallel plates of a viscoelasticity measurement instrument RDS II by Rheometric Scientific, Inc. under the conditions described above.

In detail, this specimen was heated up to 210°C and kept for 6 minutes so that an inside temperature of a chamber becomes stable at 210°C, thereafter, complex viscosity coefficients ($\eta$ *) were measured at 210°C, 190°C, and 170°C, and then a shift factor ($a_T$) was calculated from the equation described above. Then, on the basis of relation between a shift factor ($a_T$) and a measurement temperature (T), an apparent activation energy value (Ea) (kJ/mol), that is, a kneading status monitor index (M) was calculated.

**Filler dispersion index (N)**

An amount of 300gr of the unvulcanized rubber composition described above which does not contain any vulcanizing agent and any vulcanization accelerator was wound around an 8-inch open roll mill, and 1.5 phr of Sulfur as a vulcanizing agent, 0.5 phr of NOCCELER M (trade name, by Oouchi Sinko Kagaku Kogyo K.K.) and 1.0 phr of NOCCELER TT (trade name, by Oouchi Sinko Kagaku Kogyo K.K.) as vulcanization accelerators were added, and then they were kneaded.
The kneading conditions of an open roll mill were the followings;

  1) Front roll temperature/back roll temperature: 60°C/60°C
  2) Roll guide width: 40 cm
  3) Roll gap: 1 mm

After each three times cuts from both the left and the right, and 8 times rolling and recharge were done as a kneading method, an unvulcanized rubber sheet in 3 mm thickness was prepared by adjusting a roll gap at 3 mm.
Then, the unvulcanized rubber composition was vulcanized at 160°C for 8 minutes under a pressure supplied by a 50-ton press machine to make a rubber sheet in 1 mm thickness. This vulcanized rubber sheet was punched out into a couple of narrow rectangular specimen with 10 mm in width and 30 mm in length.
Regarding the narrow rectangular sample, the strain dependency of a dynamic elastic modulus was measured in the same conditions as described above by using the viscoelasticity measurement instrument RSA II made by Rheometric Scientific, Inc. described above.
Then, the strain dependency of a dynamic elastic modulus was plotted on a graph. For example, as shown in Fig.1, after finding a dynamic elastic modulus (more precisely a complex modulus) E*(a) of the vulcanized rubber sheet at 0.01% of strain ($\varepsilon$) which was in a zone where a dynamic elastic modulus (more precisely a complex modulus) (E*) had little change, and a dynamic elastic modulus (more precisely a complex modulus) E*(b) at 2% of strain ($\varepsilon$) which was in a zone where a dynamic elastic modulus (more precisely a complex modulus) had a large change, then a filler dispersion index (N) was calculated from the following equation.

$$N (\%) = (E^*(b) / E^*(a)) \times 100$$

     (More precisely, N (%) = (| E*(b) | / |E*(a)|) x 100)
The results are shown in Table 1.
Further, the unvulcanized rubber composition containing a vulcanizing agent and a vulcanization accelerator described above was used for a extrusion molding under the following conditions. Then, its die swell ratio was calculated and the surface of an extrusion molding product obtained was observed to evaluate with the following scores.

<Extrusion molding condition>

     50 mm Φ extrusion molding machine
     Width of dice opening: Φ 8 mm
     Length of dice opening: 5 mm
     Temperature of extruded resin: 80°C
     Extrusion speed of resin: 20 m/min

<Scores of extruding surface>

     5 ... Smooth surface with glaze
     4 ... Smooth surface
     3 ... Smooth but obscure surface
     2 ... Obscure surface with dents in part

1 ... Rough surface

**<u>Comparative example 1</u>**

The same composition and the same kneading duration of time (50 sec) as those of Example 1 are repeated except that the composition was kneaded under pre-heating the closed type mixer up to 170°C by steam heating.
The results are shown in Table 1.

Table 1

| | Kneading status of reference material | Examples 1 | 2 | 3 | 4 | 5 | 6 | Compar. example 1 |
|---|---|---|---|---|---|---|---|---|
| Kneading duration of time in closed type mixer [Sec.] | | | | | | | | |
|    Kneading method (A1 Method) | — | 50 | 110 | 240 | — | 360 | — | 50 |
|    Kneading method (A2 Method) | — | — | — | — | 240 | — | 360 | — |
| Kneading status monitor index (P) | | | | | | | | |
| Apparent activation energy [kJ/mol] | 118 | — | — | — | — | — | — | — |
| Kneading status monitor index (M) | | | | | | | | |
| Apparent activation energy [kJ/mol] | — | 105 | 104 | 77 | 104 | 65 | 103 | 45 |
| M／P | 1.0 | 0.89 | 0.88 | 0.65 | 0.88 | 0.55 | 0.87 | 0.38 |
| Filler dispersion index (R) | 66 | — | — | — | — | — | — | — |
| Filler dispersion index (N) | — | 30 | 55 | 59 | 59 | 63 | 63 | 23 |
|    $E^*(a) \times 10^7$ [kg/cm²] | — | 5.0 | 4.3 | 3.2 | 3.2 | 2.8 | 2.8 | 6.1 |
|    $E^*(b) \times 10^9$ [kg/cm²] | — | 1.5 | 2.4 | 1.9 | 1.9 | 1.8 | 1.8 | 1.4 |
| N／R | 1.0 | 0.45 | 0.83 | 0.90 | 0.89 | 0.95 | 0.96 | 0.35 |
| Die swell ratio [%] | 121 | 128 | 121 | 108 | 121 | 102 | 122 | 103 |
| Extruding surface | 5 | 1 | 4 | 4 | 5 | 5 | 5 | 1 |
| $T_B$ [MPa] | 15.1 | 11.2 | 13.2 | 9.4 | 14.3 | 8.5 | 15.1 | 7.3 |
| $E_B$ [%] | 620 | 450 | 560 | 450 | 580 | 430 | 610 | 385 |
| $C_s$ (70°C−22hrs) [%] | 19 | 39 | 25 | 37 | 22 | 39 | 20 | 45 |

EP 1 172 641 A1

Example 7 and Comparative Examples 2 and 3

Kneading status monitor index (P)

100 parts by weight of natural rubber (RSS#1), 48 parts by weight of HAF carbon black (Trade name: ASAHI#70, Asahi Carbon K.K.), 5 parts by weight of zinc oxide #1, 2 parts by weight of sulfur as a vulcanization agent, and 1.35 parts by weight of N-tetrabutyle-2-benzothiazolesulfenamide (Trade name: NOCCELER NS-P, by Oouchi shinko Kagaku Kogyou K.K.) were kneaded by a 8-inch open roll mill at 60°C to get an unvulcanized rubber composition.

Then, about 20 g of the unvulcanized rubber composition was sampled, pressed by a 50-ton press machine at 160°C for 6 minutes, followed by 2 minutes press with residual heat, and cooled down with water for 5 minutes. Thus, an unvulcanized rubber sheet in 2 mm thickness with a square shape of 10cm x 10cm was obtained.

This unvulcanized rubber sheet was punched out into a couple of disc specimen in 25 mm diameter for a complex viscosity coefficient measurement. Regarding this specimen, a complex viscosity coefficient ($\eta$*) was measured by using parallel plates of a viscoelasticity measurement instrument RDS II by Rheometric Scientific, Inc. under the conditions described above.

In detail, this specimen was heated up to 130°C and kept for 6 minutes so that an inside temperature of the chamber becomes stable at 130°C, thereafter, complex viscosity coefficients ($\eta$ *) were measured at 130°C, 110°C, and 90°C, and then a shift factor ($a_T$) was calculated from the equation described above. More specifically, in the case of a sequential measurement of complex viscosity coefficients ($\eta$*) at each temperatures of 130°C, 110°C, and 90°C, at first, a complex viscosity coefficient at 130°C was measured after the specimen was heated up to 130°C and kept for 6 minutes so that an inside temperature of a chamber becomes stable at 130°C, then a temperature was changed at a rate of -5°C/min from 130°C to 110°C. After the specimen was kept for 6 minutes so that an inside temperature of a chamber becomes stable at 110°C, a complex viscosity coefficient ($\eta$ *) at 110°C was measured, and then a complex viscosity coefficient ($\eta$ *) at 90°C was measured after reaching 90°C in the same manner as described above. Subsequently, on the basis of a relation between a shift factor ($a_T$) and a measurement temperature (T), an apparent activation energy value (Ea) (kJ/mol) at a reference temperature of 110°C, that is, a target kneading status monitor index (P) was calculated.

Target filler dispersion index (R)

100 parts by weight of natural rubber (RSS#1), 48 parts by weight of HAF carbon black (Trade name: ASAHI#70, Asahi Carbon K.K.), 5 parts by weight of zinc oxide #1, 2 parts by weight of sulfur as a vulcanization agent, and 1.35 parts by weight of N-tetrabutyle-2-benzothiazolesulfenamide (Trade name: NOCCELER NS-P, by Oouchi shinko Kagaku Kogyou K.K.) were kneaded by a 8-inch open roll mill at 70°C to get an unvulcanized rubber composition.

Then, the unvulcanized rubber composition was vulcanized at 160°C for 8 minutes under a pressure supplied by a 50-ton press machine to make a rubber sheet in 1 mm thickness. This vulcanized rubber sheet was punched out into a couple of narrow rectangular specimen with 10 mm in width and 30 mm in length.

Regarding the narrow rectangular sample, the strain dependency of a dynamic elastic modulus (more precisely a complex modulus) was measured in the same manner as describe before by using the viscoelasticity measurement instrument RSA II made by Rheometric Scientific, Inc. described above.

Then, the strain dependency of a dynamic elastic modulus (more precisely a complex modulus) was plotted on a graph (not shown). Then, for example, a target filler dispersion index (R) was calculated from the following equation after finding a dynamic elastic modulus (more precisely a complex modulus) E*(a) of the vulcanized rubber sheet at 0.01% of strain ($\varepsilon$) which was in a zone where a dynamic elasticmodulus (more precisely a complex modulus) (E*) had little change, and a dynamic elastic modulus (more precisely a complex modulus) E*(b) at 2% of strain ($\varepsilon$) which was in a zone where a dynamic elastic modulus (more precisely a complex modulus) had a large change.

$$R \, (\%) = (E^*(b)/ \, E^*(a)) \times 100$$

(More precisely, R (%) = (|E*(b) | / |E*(a)|) x 100)

Kneading status monitor index (M)

100 parts by weight of natural rubber (RSS#1), 48 parts by weight of HAF carbon black (Trade name: ASAHI#70, Asahi Carbon K.K.), 5 parts by weight of zinc oxide #1, 2 parts by weight of sulfur, and 1.35 parts by weight of N-tetrabutyle-2-benzothiazolesulfenamide (Trade name: NOCCELER NS-P, by Oouchi shinko Kagaku Kogyou K.K.) were kneaded at a kneading initial temperature of 50°C by using a Kobe Seikosyo 1.7 liter BB2 type Banbury mixer which is a closed type mixer (Banbury mixer, hereafter). Under these kneading conditions, an unvulcanized rubber composition was obtained.

The kneading method used in Example 7 by using the closed type mixer described above was carried out in accordance with the kneading method (A1 method) specified in JIS K6299 and, its kneading duration of time was 180 sec. Also, the temperature of the composition immediately after its discharge from the Banbury mixer was 115°C.

The kneading method with Banbury mixer described above was carried out in Comparative Example 2 and Comparative Example 3 in accordance with the kneading method (Al method) specified at JIS K6299, and their kneading durations of time were 40 sec and 480 sec, respectively. Also, the temperatures of the composition immediately after its discharge from the Banbury mixer were 75°C and 155°C, respectively.

Then, about 20 g of the unvulcanized rubber composition was sampled, pressed by a 50-ton press machine at 160°C for 6 minutes, followed by 2 minutes press with residual heat, and cooled down with water for 5 minutes. Thus, an unvulcanized rubber sheet in 2 mm thickness with a square shape of 10cm x 10cm was obtained.

This unvulcanized rubber sheet was punched out into a couple of disc specimen in 25 mm diameter for a complex viscosity coefficient measurement. Regarding this specimen, a complex viscosity coefficient ($\eta^*$) was measured by using parallel plates of a viscoelasticity measurement instrument RDS II by Rheometric Scientific, Inc. under the conditions described above.

In detail, this specimen was heated up to 130°C and kept for 6 minutes so that an inside temperature of the chamber becomes stable at 130°C, thereafter, complex viscosity coefficients ($\eta^*$) were measured at 130°C, 110°C, and 90°C, and then a shift factor ($a_T$) was calculated from the equation described above. Subsequently, on the basis of the relation between a shift factor ($a_T$) and a measurement temperature (T), an apparent activation energy value (Ea) (kJ/mol) at a reference temperature of 100°C, that is, a kneading status monitor index (M) was calculated.

Filler dispersion index (N)

100 parts by weight of natural rubber (RSS#1), 48 parts by weight of HAF carbon black ( [Trade name: ASAHI#70, Asahi Carbon K.K.], 5 parts by weight of zinc oxide #1 were kneaded at a kneading initial temperature of 50°C by using a Kobe Seikosyo 1.7 liter BB2 type Banbury mixer which is a closed type mixer (Banbury mixer, hereafter). Under these kneading conditions, an unvulcanized rubber composition not containing any vulcanizing agent and any vulcanization accelerator was obtained.

Then, the unvulcanized rubber composition was taken out from a closed type mixer, and an amount of 153 parts by weight of it was wound around an 8-inch open roll mill, and 2 parts by weight of sulfur as a vulcanizing agent and 1.35 parts by weight of N-tetrabutyle-2-benzothiazole-sulfenamide (Trade name: NOCCELER NS-P, by Oouchi shinko Kagaku Kogyou K.K.) as vulcanization accelerators were added, and then they were kneaded.

The kneading conditions of an open roll mill were the followings;

1) Front roll temperature/back roll temperature: 60°C/60°C
2) Roll guide width: 40 cm
3) Roll gap: 1 mm

After each three times cuts from both the left and the right, and eight times rolling and recharge were done as a kneading method, an unvulcanized rubber sheet in 3 mm thickness was prepared by adjusting a roll gap at 3 mm.

Then, the unvulcanized rubber composition was vulcanized at 160°C for 8 minutes under a pressure supplied by a 50-ton press machine to make a rubber sheet in 1 mm thickness. This vulcanized rubber sheet was punched out into a couple of narrow rectangular specimen with 10 mm in width and 30 mm in length.

Regarding the narrow rectangular sample, the strain dependency of a dynamic elastic modulus (more precisely a complex modulus) was measured in the same conditions as described above by using the viscoelasticity measurement instrument RSA II made by Rheometric Scientific, Inc. described above.

Then, the strain dependency of a dynamic elastic modulus (more precisely a complex modulus) was plotted on a graph (not shown). For example, a filler dispersion index (N) was calculated from the following equation after finding a dynamic elastic modulus (more precisely a complex modulus) E*(a) of the vulcanized rubber sheet at 0.01% of strain ($\varepsilon$) which was in a zone where a dynamic elastic modulus (more precisely a complex modulus) (E*) had little change, and a dynamic elastic modulus (more precisely a complex modulus) E*(b) at 2% of strain ($\varepsilon$) which was in a zone where a dynamic elastic modulus (more precisely a complex modulus) had a large change.

$$N(\%)=(E^*(b) / E^*(a)) \times 100$$

(More precisely, N (%) = (|E*(b) | / |E*(a)|) x 100)

The results are shown in Table 2.

Table 2

| | Kneading status of reference material | Example | Comparative examples | |
|---|---|---|---|---|
| | | 7 | 2 | 3 |
| Formulation of rubber composition [parts by weight] | | | | |
| Natural rubber R S S # 1 | 100 | 100 | 100 | 100 |
| H A F carbon black | 48 | 48 | 48 | 48 |
| Zinc oxide | 5 | 5 | 5 | 5 |
| Vulcanization accelerator | 1.35 | 1.35 | 1.35 | 1.35 |
| Sulfur | 2 | 2 | 2 | 2 |
| Activation energy (P) [kJ/mol] | 52 | | | |
| Activation energy (M) [kJ/mol] | - | 52 | 52 | 25 |
| M/P | | 1.0 | 1.0 | 0.48 |
| Filler dispersion index (R) | 91 | | | |
| Filler dispersion index (N) | | 89 | 72 | 91 |
| $E^*(a) \times 10^7$ [kg/cm$^2$] | 5.10 | 4.90 | 4.80 | 5.10 |
| $E^*(b) \times 10^7$ [kg/cm$^2$] | 5.60 | 5.50 | 6.70 | 5.60 |
| N/R | | 0.98 | 0.79 | 1.0 |
| $T_B$ [MPa] | 26.8 | 27.8 | 19.1 | 15.2 |
| $E_B$ [%] | 510 | 520 | 480 | 320 |

## Claims

1. A kneading status evaluation method for a rubber composition (I) containing at least a rubber (A) and a filler (B), which comprises the steps of;

(1) a complex modulus measurement step to measure a complex modulus of E*(a) at a given strain ε a and a complex modulus E*(b) at a given strain ε b differing from the strain ε a,
(2) a filler dispersion index calculation step to calculate a filler dispersion index (N) of the rubber composition (I) according to the following equation;

$$\text{Filler dispersion index (N)} = |E^*(a)|/|E^*(b)|$$

where complex elastic moduli E*(a) and E*(b) are obtained at the complex modulus measurement step (1), and
(3) a comparison step to compare a predetermined target filler dispersion index (R) with the filler dispersion index (N) calculated in the filler dispersion index calculation step (2).

2. The evaluation method as claimed in claim 1, wherein the target filler dispersion index (R) is a complete target filler dispersion index (NO) obtained through the complex modulus measurement step (1) and the filler dispersion index calculation step (2) after a rubber composition having the same formulation as that of the rubber composition (I) is substantially kneaded to achieve a practically complete dispersion.

3. The evaluation method as claimed in claim 2, wherein the practically complete dispersion is achieved by kneading with an open roll mill.

4. A kneading status evaluation method for a rubber composition (I) containing at least a rubber (A) and a filler (B), which comprises the steps of;

(1') a dynamic elastic modulus measurement step to measure a dynamic elastic modulus E' (a) at a given

strain $\varepsilon$ a and a dynamic elastic modulus E' (b) at a given strain $\varepsilon$ b differing from the strain $\varepsilon$ a,
(2') a filler dispersion index calculation step to calculate a filler dispersion index (N') of the rubber composition (I) according to the following equation;

$$\text{Filler dispersion index (N') = E'(a) /E'(b)}$$

where the dynamic elastic moduli E'(a) and E'(b) are obtained at the dynamic elastic modulus measurement step (1'), and
(3') a comparison step to compare a predetermined target filler dispersion index (R') with the filler dispersion index (N') calculated in the filler dispersion index calculation step (2').

5.  The evaluation method as claimed in claim 4, wherein the target filler dispersion index (R') is a complete target filler dispersion index (NO') obtained through the dynamic elastic modulus measurement step (1') and the filler dispersion index calculation step (2') after a rubber composition having the same formulation as that of the rubber composition (I) is substantially kneaded to achieve a practically complete dispersion.

6.  The evaluation method as claimed in claim 5, wherein the practical complete dispersion is achieved by kneading with an open roll mill.

7.  A manufacturing method for a rubber composition utilizing the kneading status evaluation methods for a rubber composition according to any one of claims 1 to 6.

8.  The manufacturing method for a rubber composition as claimed in claim 7, which further comprises a feedback step (4) or (4') to control kneading conditions of the rubber composition (I) by means of adjusting a value of filler dispersion index (N)/target filler dispersion index (R) to be a certain numeric range, or a value of filler dispersion index (N')/target filler dispersion index (R') to be a certain numeric range according to the result from the comparison step (3) or (3').

9.  The manufacturing method for a rubber composition as claimed in claim 8, wherein the numeric range of the value of filler dispersion index (N)/target filler dispersion index (R) (where $|E^* \text{ (a) }| \leqq | E^* \text{ (b) }|$) or the value of filler dispersion index (N')/target filler dispersion index (R') is 0.8 to 1.0.

10. A kneading status evaluation method for a rubber composition (I) containing at least a rubber (A) and a filler (B), which comprises the steps of:

    (5) a complex viscosity coefficient measurement step to measure a complex viscosity coefficient $\eta$ * of the rubber composition (I) under at least two different temperatures,
    (6) a kneading status monitor index calculation step to calculate a kneading status monitor index (M) of the rubber composition (I) according to the following equation;

$$| \eta^*(T)| = A \exp (-M/ RT)$$

    where $\eta$ *: complex viscosity coefficient, A: proportional constant, R: gas constant, and T: measuring temperature ($^\circ$K), that shows a temperature dependency of the complex viscosity coefficient $\eta$ * obtained at the complex viscosity coefficient measurement step (5), and
    (7) a comparison step to compare a predetermined target kneading status monitor index (P) with the kneading status monitor index (M) calculated in the kneading status monitor index calculation step (6).

11. The evaluation method as claimed in claim 10, wherein the target kneading status monitor index (P) is a complete target kneading status monitoring index (M0) obtained through the complex viscosity coefficient measurement step (5) and the kneading status monitor index calculation step (6) after a rubber composition having the same proportion as that of the rubber composition (I) is substantially kneaded to achieve a practically complete dispersion.

12. The evaluation method as claimed in claim 11, wherein the practically complete dispersion is achieved by kneading with an open roll mill.

**13.** A kneading status evaluation method for a rubber composition (I) containing at least a rubber (A) and a filler (B), which comprises the steps of:

(5') a viscosity coefficient measurement step to measure a real viscosity coefficient $\eta'$ as a real part of complex viscosity coefficient $\eta *$ of the rubber composition (I) under at least two different temperatures,
(6') a kneading status monitor index calculation step to calculate a kneading status monitor index (M') of the rubber composition (I) according to the following equation;

$$\eta'(T) = A \exp(-M'/RT)$$

where A: proportional constant, R: gas constant, and T: measuring temperature ($^\circ$K), that shows a temperature dependency of a real viscosity coefficient $\eta'$ obtained as a real part of complex viscosity coefficient $\eta*$ at the viscosity coefficient measurement step (5'), and
(7') a comparison step to compare a predetermined target kneading status monitor index (P') with the kneading status monitor index (M') obtained in the kneading status monitor index calculation step (6').

**14.** The evaluation method as claimed in claim 13, wherein the target kneading status monitor index (P') is a complete target kneading status monitor index (M0') obtained through the viscosity coefficient measurement step (5') and the kneading status monitor index calculation step (6') after a rubber composition having the same formulation as that of the rubber composition (I) is substantially kneaded to achieve a practically complete dispersion.

**15.** The evaluation method as claimed in claim 14, wherein the practical complete dispersion is achieved by kneading with an open roll mill.

**16.** A manufacturing method for a rubber composition utilizing the kneading status evaluation methods for a rubber composition according to any one of claims 10 to 15.

**17.** The manufacturing method for a rubber composition as claimed in claim 16, which further comprises a feedback step (8) or (8') to control kneading conditions of the rubber composition (I) by means of adjusting a value of kneading status monitor index (M)/target kneading status monitor index (P) to be a certain numeric range, or a value of kneading status monitor index (M')/target kneading status monitor index (P') to be a certain numeric range according to the result from the comparison step (7) or (7').

**18.** The manufacturing method for a rubber composition as claimed in claim 17, wherein the numeric range of the value of kneading status monitor index (M) /target kneading status monitor index (P) or the value of kneading status monitor index (M')/target kneading status monitor index (P') is 0.85 to 1.0.

Fig. 1

EP 1 172 641 A1

Fig. 2

Fig. 3

(a)

at 190°C

at 170°C

$\eta *$
(Pa)

at 210°C

Frequency (Hz)

(b)

$10^1$

$a_T$

$E_a$

$10^0$

$10^{-1}$

170    190    210

Temperature (°C)

## INTERNATIONAL SEARCH REPORT

| International application No. |
|---|
| PCT/JP01/00634 |

**A. CLASSIFICATION OF SUBJECT MATTER**
Int.Cl$^7$  G01N19/00, G01N11/00, G01N33/44
C08L21/00, C08J3/20, C08C4/00

According to International Patent Classification (IPC) or to both national classification and IPC

**B. FIELDS SEARCHED**

Minimum documentation searched (classification system followed by classification symbols)
Int.Cl$^7$  G01N19/00, G01N11/00, G01N33/44
C08L21/00, C08J3/20, C08C4/00

Documentation searched other than minimum documentation to the extent that such documents are included in the fields searched
Jitsuyo Shinan Koho        1922-1996    Toroku Jitsuyo Shinan Koho  1994-2001
Kokai Jitsuyo Shinan Koho  1971-2001    Jitsuyo Shinan Toroku Koho  1996-2001

Electronic data base consulted during the international search (name of data base and, where practicable, search terms used)
JICST FILE(JOIS)
WPI/L(QUESTEL)

**C. DOCUMENTS CONSIDERED TO BE RELEVANT**

| Category* | Citation of document, with indication, where appropriate, of the relevant passages | Relevant to claim No. |
|---|---|---|
| A | JP, 63-95243, A (Ube Industries, Ltd.), 26 April, 1988 (26.04.88), Full text   (Family: none) | 1-18 |
| P,X | Ichino, Nakahama, Matsunaga, Kamo, "Shin Konren Shihyou No Kentou (1)-(3)", Nippon rubber Kyoukai Kenkyu Happyou Kouenkai,(Japan), May, 2000, pp.55-57 | 1-18 |

☐ Further documents are listed in the continuation of Box C.    ☐ See patent family annex.

| | | |
|---|---|---|
| * | Special categories of cited documents: | |
| "A" | document defining the general state of the art which is not considered to be of particular relevance | |
| "E" | earlier document but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search 17 April, 2001 (17.04.01) | Date of mailing of the international search report 01 May, 2001 (01.05.01) |
|---|---|
| Name and mailing address of the ISA/ Japanese Patent Office | Authorized officer |
| Facsimile No. | Telephone No. |

Form PCT/ISA/210 (second sheet) (July 1992)